# EUROPÄISCHE PATENTANMELDUNG

(11) **EP 1 972 309 A1**
(43) Veröffentlichungstag der Anmeldung: **24.09.2008**
(21) Anmeldenummer: 08152575.0
(22) Anmeldetag: 11.03.2008
(51) Int. Cl.: A61F 2/44

(54) **Implantat mit Röntgenmarker**

(30) Priorität: 19.03.2007 DE 102007014285
(71) Anmelder: Aesculap AG, 78532 Tuttlingen (DE)
(72) Erfinder: Wagner, Cécile, 78532 Tuttlingen (DE); Zeller, Richard, 78532 Tuttlingen (DE); Beger, Jens, 78532 Tuttlingen (DE)
(74) Vertreter: Hoeger, Stellrecht & Partner Patentanwälte

(57) **Zusammenfassung**

Um ein Implantat zum Ersetzen eines Wirbelkörpers oder zum Einsetzen in einen Zwischenwirbelraum zwischen benachbarte Wirbelkörper einer menschlichen oder tierischen Wirbelsäule, mit einer ersten Anlagefläche (26) zum Anlegen an eine Gelenkfläche eines ersten Wirbelkörpers und mit einer zweiten Anlagefläche (28) zum Anlegen an eine Gelenkfläche eines zweiten Wirbelkörpers, wobei das Implantat mindestens teilweise aus einem röntgentransparenten Material hergestellt ist und mindestens einen Röntgenmarker umfasst, so zu verbessern, dass es einfacher hergestellt werden kann, wird vorgeschlagen, dass der mindestens eine Röntgenmarker (42) in Form einer Metall als Markermaterial enthaltenden, mindestens teilweisen Markerbeschichtung (44) einer Seitenfläche des Implantats, welche relativ zur ersten und/oder zweiten Anlagefläche um einen Neigungswinkel geneigt ist, ausgebildet ist.

## Beschreibung

Die vorliegende Erfindung betrifft ein Implantat zum Ersetzen eines Wirbelkörpers oder zum Einsetzen in einen Zwischenwirbelraum zwischen benachbarte Wirbelkörper einer menschlichen oder tierischen Wirbelsäule, mit einer ersten Anlagefläche zum Anlegen an eine Gelenkfläche eines ersten Wirbelkörpers und mit einer zweiten Anlagefläche zum Anlegen an eine Gelenkfläche eines zweiten Wirbelkörpers, wobei das Implantat mindestens teilweise aus einem röntgentransparenten Material hergestellt ist und mindestens einen Röntgenmarker umfasst.

Bei einer klassischen Behandlung eines Bandscheibenvorfalls wird eine zwischen benachbarten Wirbelkörpern in einem auch als Bandscheibenfach bezeichneten Zwischenwirbelraum angeordnete Bandscheibe ganz oder teilweise entfernt und das die beiden benachbarten Wirbelkörper umfassende Wirbelsäulensegment fusioniert. Hierzu wird üblicherweise ein Distanzhalter, welcher auch als "Cage" bezeichnet wird, in das Bandscheibenfach eingesetzt. Dadurch kann einerseits eine Höhe des Segments, also ein Abstand zwischen Gelenkflächen benachbarter Wirbelkörper, erhalten und andererseits können die beiden Wirbelkörper fest miteinander verbunden werden, so dass insbesondere auch Knochen in dem Bandscheibenfach wachsen kann.

In den letzten Jahren wurden Zwischenwirbelimplantate in Form von Distanzhaltern auch aus röntgendurchlässigen Materialien bekannt. Dadurch ist es möglich, einen Knochendurchbau des Implantats durch Röntgen- oder CT-Aufnahmen sichtbar zu machen. Nachteilig bei röntgendurchlässigen Werkstoffen von Zwischenwirbelimplantaten oder auch Implantaten zum Ersetzen eines Wirbelkörpers ist, dass zur Überprüfung einer Positionierung der Implantate zusätzliche Röntgenmarker am Implantat vorgesehen werden müssen, die es ermöglichen, eine Lage des Implantats nach einer Implantation zum Beispiel auf einem Röntgenbild zu überprüfen. Derartige Röntgenmarker am Implantat auszubilden ist aufwendig und teuer.

Es ist daher Aufgabe der vorliegenden Erfindung, ein Implantat der eingangs beschriebenen Art so zu verbessern, dass es einfacher hergestellt werden kann.

Diese Aufgabe wird bei einem Implantat der eingangs beschriebenen Art erfindungsgemäß dadurch gelöst, dass der mindestens eine Röntgenmarker in Form einer Metall als Markermaterial enthaltenden, mindestens teilweisen Markerbeschichtung einer Seitenfläche des Implantats, welche relativ zur ersten und/oder zweiten Anlagefläche um einen Neigungswinkel geneigt ist, ausgebildet ist.

Eine Markerbeschichtung aufzubringen ist deutlich einfacher und kostengünstiger als in bekannter Weise Stifte oder Kugeln am Implantat anzubringen. Zudem kann die Markerbeschichtung, je nach Wahl eines Beschichtungsmaterials, auch ein An- oder Einwachsen von Knochen am oder ins Implantat verbessern. Ferner kann beispielsweise auf einer Röntgenaufnahme einer Wirbelsäule in Seitenansicht eine Position der Markerbeschichtung einer Seitenfläche des Implantats optimal gesehen werden. Insbesondere ist die Markerbeschichtung derart ausgebildet, dass sie eine oder mehrere Seitenflächen des Implantats nur teilweise bedeckt. Selbstverständlich kann auch eine komplette Seitenfläche bedeckt sein, so dass diese insgesamt als Röntgenmarker dient.

Besonders kostengünstig in der Herstellung wird das Implantat, wenn das röntgentransparente Material ein Kunststoff ist. Kunststoffe haben zudem den Vorteil, dass eine an Knochen angenäherte Druckfestigkeit einstellbar ist, im Vergleich zu einem aus Metall hergestellten Implantat also eine gewisse Elastizität des Implantats vorgebbar ist.

Vorzugsweise ist oder enthält der Kunststoff Polyetheretherketon (PEEK). Ein derartiger Kunststoff ist bereits als Implantationsmaterial zugelassen.

Günstigerweise weist der Neigungswinkel einen Wert in einem Bereich von 45° bis 135° auf. So können insbesondere durch die Markerbeschichtung vorgegebene definierte Strukturen in einer Röntgenaufnahme der Wirbelsäule von der Seite besonders gut erkannt werden.

Vorteilhaft ist es, wenn der Neigungswinkel einen Wert in einem Bereich von 70° bis 110° aufweist. Vorzugsweise beträgt der Neigungswinkel 90°. Dies bedeutet, dass eine oder mehrere Seitenflächen auch senkrecht zu den Anlageflächen ausgebildet sein können.

Um insbesondere eine Orientierung des Implantats auf einer Röntgenaufnahme eindeutig angeben zu können, ist es günstig, wenn der mindestens eine Röntgenmarker in Form eines regelmäßigen oder unregelmäßigen Musters ausgebildet ist. Außerdem wird so die Herstellung des Implantats vereinfacht.

Vorzugsweise ist der mindestens eine Röntgenmarker in Form mindestens eines Buchstabens, einer Zahl und/oder eines Codes ausgebildet. Denkbar wären insbesondere auch Strichcodes, beispielsweise Barcodes, mit denen zum einen eine Lage und/oder Orientierung des Implantats im Körper bestimmbar und zum anderen auch noch nach Implantation jederzeit Angaben über beispielsweise Art, Typ, Chargennummer, Hersteller und/oder Material, aus dem das Implantat hergestellt ist, oder dergleichen, ablesbar sind. Der Röntgenmarker kann also gleichzeitig als optisches Speicherelement für Implantatcharakteristika genutzt werden.

Um eine Position des Implantats im Körper des Patienten noch besser und genauer bestimmen zu können, sind vorteilhafterweise mehrere Röntgenmarker vorgesehen.

Gemäß einer bevorzugten Ausführungsform der Erfindung können die mehreren Röntgenmarker mindestens teilweise auf unterschiedlichen Seitenflächen und/oder unterschiedlichen Seiten des Implantats vorgesehen sein. So kann eine Position und/oder eine Orientierung des Implantats im Körper des Patienten unabhängig oder im Wesentlichen unabhängig von einer Aufnahmerichtung quer zu einer Körperlängsachse stets präzise bestimmt werden.

Besonders günstig ist es, wenn das Markermaterial Titan enthält. So lässt sich die Gefahr einer Abstoßung des Implantats für den Patienten minimieren.

Besonders günstig ist es, wenn die Markerbeschichtung aus Reintitan hergestellt ist. So lässt sich eine Abstoßungsreaktion praktisch völlig ausschließen.

Besonders einfach lässt sich die Beschichtung insbesondere auf ein röntgendurchlässiges Material aufbringen, wenn sie durch Kaltgasspritzen hergestellt ist. Besonders niedrige Prozesstemperaturen ermöglichen es, metallische Beschichtungen auch auf Kunststoffen mit niedriger Schmelztemperatur oder einem niedrigen Fließtemperaturbereich aufzubringen.

Damit insbesondere ein röntgendurchlässiges Material, beispielsweise ein Kunststoff, nicht durch das Aufbringen der Markerbeschichtung beschädigt wird, ist es vorteilhaft, wenn eine Teilchengeschwindigkeit durch Kaltgasspritzen aufgebrachter Pulverteilchen des Markermaterials in einem Bereich von 600 m/s bis 1000 m/s liegt. Werden die Pulverteilchen des Markermaterials mit derartigen Geschwindigkeiten auf das zu beschichtende Implantat zu bewegt, wird eine optimale Verbindung der Pulverteilchen mit dem röntgentransparenten Material ermöglicht und eine dauerhafte Verbindung der Beschichtung und des Implantats sichergestellt.

Um insbesondere ein aus einem Kunststoff hergestelltes Implantat beim Aufbringen der Markerbeschichtung nicht zu schädigen, ist es günstig, wenn eine Strahltemperatur beim Kaltgasspritzen in einem Bereich von 250°C bis 700°C liegt. Vorzugsweise liegt die Strahltemperatur in einem Bereich von 250°C bis 500°C.

Vorzugsweise weist die Markerbeschichtung eine Dicke in einem Bereich von 0,1 mm bis 0,3 mm auf. So kann eine ausreichende Röntgenstrahlenundurchlässigkeit erzeugt werden, so dass die Markerbeschichtung auf einem Röntgenbild gut erkennbar ist.

Um zu verhindern, dass sich das Implantat bis zu einer endgültigen Fusion relativ zu den Wirbelkörpern bewegt, ist es vorteilhaft, wenn die erste und/oder die zweite Anlagefläche strukturiert sind.

Besonders vorteilhaft ist es, wenn die erste und/oder die zweite Anlagefläche Vorsprünge und/oder Ausnehmungen aufweisen. Diese können insbesondere eine Strukturierung bilden und vorzugsweise in Form von Rinnen, Zahnungen, Erhöhungen oder dergleichen ausgebildet sein, die zumindest teilweise in eine Oberfläche eines Wirbelkörpers eingreifen können, um eine Relativbewegung bis zu einer endgültigen Fusion der benachbarten Wirbelkörper miteinander zu verhindern.

Gemäß einer bevorzugten Ausführungsform der Erfindung kann vorgesehen sein, dass das Implantat ein erstes Anlageelement und ein zweites Anlageelement umfasst, dass das erste Anlageelement die erste Anlagefläche umfasst und dass das zweite Anlageelement die zweite Anlagefläche umfasst. Durch entsprechende Wahl der Anlageelemente, sowohl was deren Form als auch das Material, aus dem sie hergestellt sind, anbelangt, können optimierte Implantate für jeden Verwendungszweck ausgebildet werden, insbesondere sowohl als Zwischenwirbelimplantat, also in Form einer künstlichen Bandscheibe, oder als Wirbelkörperimplantat zum vollständigen oder teilweisen Ersetzen eines degenerierten und resezierten Wirbelkörpers.

Besonders einfach lässt sich eine Verblockung benachbarter Wirbelkörper erreichen, wenn das erste und das zweite Anlageelement unbeweglich miteinander verbunden sind.

Eine Stabilität des Implantats kann weiter erhöht werden, wenn es einen Grundkörper umfasst, welcher das erste und das zweite Anlageelement umfasst und einstückig ausgebildet ist.

Das Einwachsen von Knochen in das Implantat wird vereinfacht und beschleunigt, wenn der Grundkörper teilweise hohl ist und/oder mindestens eine Ausnehmung aufweist.

Insbesondere zur Ausbildung künstlicher Bandscheiben ist es vorteilhaft, wenn das erste und das zweite Anlageelement relativ zueinander beweglich gelagert sind.

Vorzugsweise sind das erste und das zweite Anlageelement relativ zueinander verschwenkbar und/oder verschiebbar gelagert. Eine verschwenkbare Lagerung kann insbesondere um eine Schwenkachse vorgesehen sein. Denkbar wäre auch eine verschwenkbare Lagerung um ein Gelenkzentrum, beispielsweise könnte eine gelenkige Lagerung in Form eines Kugelgelenks vorgesehen sein.

Zur Verbesserung eines Ein- oder Anwachsens des Implantats an einen Wirbelkörper kann es vorteilhaft sein, wenn die erste und/oder die zweite Anlagefläche mindestens teilweise mit einer osteointegrativen Beschichtung versehen sind. Vorzugsweise sind die erste und/oder die zweite Anlagefläche vollständig mit einer osteointegrativen Beschichtung versehen. Diese Beschichtung kann insbesondere gleichzeitig mit der Markerbeschichtung aufgebracht werden, wenn diese aus demselben Material hergestellt sind. Dadurch vereinfacht sich eine Fertigung insbesondere von Implantaten, die eine osteointegrative Beschichtung aufweisen.

Das Einwachsen von Knochen wird erleichtert und damit eine dauerhafte Verbindung des Implantats mit dem benachbarten Wirbelkörper, wenn die osteointegrative Beschichtung porös ist.

Zur Vermeidung von Abstoßungsreaktionen ist es vorteilhaft, wenn die osteointegrative Beschichtung eine Titan enthaltende Beschichtung ist.

Besonders günstig ist es jedoch, wenn die osteointegrative Beschichtung eine Rein-Titan-Beschichtung ist. Eine solche Beschichtung weist besonders gute Eigenschaften für das An- und Einwachsen von Knochen in das Implantat auf.

Besonders einfach lässt sich die osteointegrative Beschichtung aufbringen, insbesondere auf aus Kunststoffen hergestellten Implantaten, wenn sie durch Kaltgasspritzen hergestellt ist. Sie weist zudem dieselben Vorteile auf, wie eine durch Kaltgasspritzen hergestellte Markerbeschichtung.

Für eine stabile, dauerhafte und das Einwachsen von Knochen erleichternde Beschichtung ist es vorteilhaft, wenn die osteointegrative Beschichtung eine Dicke in einem Bereich von 0,2 mm bis 0,5 mm aufweist.

Günstig ist es, wenn die osteointegrative Beschichtung dicker ist als die Markerbeschichtung. Diese Ausgestaltung ermöglicht es insbesondere, das Implantat komplett zu beschichten, beispielsweise mit einer dicken osteointegrativen Beschichtung auf den Anlageflächen zu versehen und alle anderen Seitenflächen des Implantats mit einer dünneren Markerbeschichtung. Man könnte dann in einer Röntgenaufnahme trotzdem einen in das Implantat eingewachsenen Knochen erkennen.

Die nachfolgende Beschreibung bevorzugter Ausführungsformen der Erfindung dient im Zusammenhang mit der Zeichnung der näheren Erläuterung. Es zeigen:
- Figur 1:: eine Draufsicht auf ein in einen Zwischenwirbelraum eingesetztes Implantat; und
- Figur 2:: eine schematische Darstellung einer Röntgenaufnahme mit Blickrichtung in Richtung der Pfeile in Figur 1.

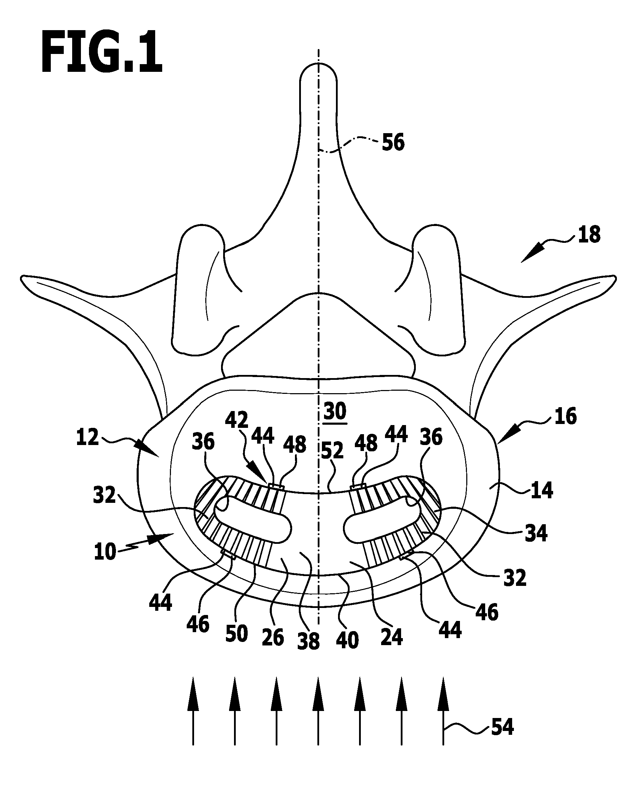

In Figur 1 ist beispielhaft ein insgesamt mit dem Bezugszeichen 10 versehenes Implantat zum Einsetzen in einen Zwischenwirbelraum 12 zwischen benachbarte Wirbelkörper 14 zweier Wirbel 16 einer Wirbelsäule 18 eines Patienten dargestellt.

Das Implantat 10 umfasst ein erstes Anlageelement 20 und ein zweites Anlageelement 22, die unbeweglich miteinander verbunden sind, und zwar durch einen Grundkörper 24, welcher das erste und zweite Anlageelement 20 und 22 umfasst und einstückig aus einem röntgentransparenten Material hergestellt ist. Das röntgentransparente Material ist ein Kunststoff, vorzugsweise Polyetheretherketon (PEEK). Das erste Anlageelement 20 umfasst eine erste Anlagefläche 26 zum Anlegen an eine Gelenkfläche eines ersten Wirbelkörpers 14. Das zweite Anlageelement 22 umfasst eine zweite Anlagefläche 28 zum Anlegen an eine Gelenkfläche 30 eines zweiten Wirbelkörpers 14.

Sowohl die erste Anlagefläche 26 als auch die zweite Anlagefläche 28 sind strukturiert. Sie weisen jeweils zwei Verzahnungsbereiche 32 auf, mit von der jeweiligen Anlagefläche 26 beziehungsweise 28 weg weisenden streifenförmig ausgebildeten Zähnen 34.

Des Weiteren weist das in einer Draufsicht in etwa nierenförmig geformte Implantat 10 zwei langlochartige Durchbrechungen 36 auf, die beide Anlageflächen 26 und 28 durchsetzen und eine Durchtrittsrichtung definieren, welche senkrecht zu den Anlageflächen 26 und 28, die parallel zueinander verlaufen, orientiert ist. Der Grundkörper 24 ist somit teilweise hohl oder weist zumindest eine Ausnehmung in Form einer Durchbrechung 36 auf.

Die erste Anlagefläche 26 und die zweite Anlagefläche 28 sind jeweils mit einer osteointegrativen Beschichtung 38 versehen. Diese Beschichtung 38 ist vorzugsweise porös. Des Weiteren kann sie Titan enthalten oder in Form einer Rein-Titan-Beschichtung ausgebildet sein. Sie weist eine Dicke in einem Bereich von 0,2 mm bis 0,5 mm auf, vorzugsweise in einem Bereich von 0,3 mm bis 0,4 mm. Die Beschichtung 38 ist durch Kaltgasspritzen hergestellt, wobei bei der Herstellung eine Teilchengeschwindigkeit der durch das Kaltgasspritzen aufgebrachten Pulverteilchen in einem Bereich von 600 m/s bis 1000 m/s liegt. Ferner liegt eine Strahltemperatur beim Kaltgasspritzen in einem Bereich von 250°C bis 700°C, vorzugsweise in einem Bereich von 250°C bis 500°C.

Die osteointegrative Beschichtung erleichtert das An- beziehungsweise Einwachsen von Knochen an dem beziehungsweise in das Implantat 10.

Das Implantat 10 weist auf Grund seiner nierenförmigen Gestalt nur eine einzige, zusammenhängende Seitenfläche 40 auf, welche bei dem in Figur 1 gezeigten Ausführungsbeispiel senkrecht oder im Wesentlichen senkrecht zu den Anlageflächen 26 und 28 orientiert ist. Das heißt, sie ist relativ zu den Anlageflächen 26 und 28 um einen Neigungswinkel geneigt, welcher einen Wert von 90° aufweist. Die Seitenfläche 40, es können auch mehrere Seitenflächen vorgesehen sein, und zwar je nach Gestalt des Implantats 10, kann relativ zu den Anlageflächen 26 und 28 jedoch auch um einen Neigungswinkel geneigt sein, welcher einen Wert in einem Bereich von 45° bis 135° aufweist, vorzugsweise in einem Bereich von 70° bis 110°.

Das Implantat 10 umfasst ferner einen Röntgenmarker 42, welcher in Form einer Markerbeschichtung 44 ausgebildet ist, welche vier Streifen 46 beziehungsweise 48 umfasst, wobei die beiden Streifen 46 auf einer nach vorne weisenden Seite 50 des Implantats 10 senkrecht zu den Anlageflächen 26 und 28 verlaufend aufgebracht sind, die beiden Streifen 48 auf einer nach hinten weisenden Seite 52. Ein Abstand zwischen den beiden Streifen 46 auf der Seite 50 ist größer als ein Abstand zwischen den Streifen 48 auf der Seite 52. Alle Streifen 46 und 48 verlaufen senkrecht zu den Anlageflächen 26 und 28.

Der Röntgenmarker 42 ist somit in Form eines regelmäßigen Musters ausgebildet. Im vorliegenden Fall ist der Röntgenmarker 42 codeartig ausgebildet, wobei die Streifen 46 und 48 Bestandteile des Codes bilden. Alternativ zu den Streifen 46 und 48 können auch direkt Buchstaben, Zahlen oder andere beliebige geometrische Figuren als Beschichtung aufgebracht werden. Selbstverständlich können auch mehrere Röntgenmarker vorgesehen sein, wobei jeder der Streifen 46 und 48 auch als individueller Röntgenmarker betrachtet werden kann.

Der Röntgenmarker 42 ist vorzugsweise aus einem Metall, insbesondere aus einem Titan enthaltenden Metall gebildet. Bei dem in den Figuren dargestellten Ausführungsbeispiel ist die Markerbeschichtung aus Reintitan hergestellt. Sie ist ferner durch Kaltgasspritzen aufgebracht, wobei eine Teilchengeschwindigkeit der durch Kaltgasspritzen aufgebrachten Pulverteilchen des Markermaterials in einem Bereich von 600 m/s bis 1000 m/s liegt. Eine Strahltemperatur beim Kaltgasspritzen der Markerbeschichtung 44 liegt in einem Bereich von 250°C bis 700°C, vorzugsweise in einem Bereich von 250°C bis 500°C. Eine Dicke der Markerbeschichtung 44 liegt in einem Bereich von 0,1 mm bis 0,3 mm. Vorzugsweise ist die Markerbeschichtung 44 dünner als die osteointegrative Beschichtung 38.

Herstellungstechnisch können sowohl die Markerbeschichtung 44 als auch die osteointegrative Beschichtung 38 gleichzeitig aufgebracht werden. Dies vereinfacht insgesamt die Herstellung des Implantats, denn es müssen nicht, wie bei bekannten Implantaten, welche Röntgenmarker aufweisen, Röntgenmarker in Form von Stiften oder Kugeln nachträglich in den Grundkörper des Implantats eingesetzt werden.

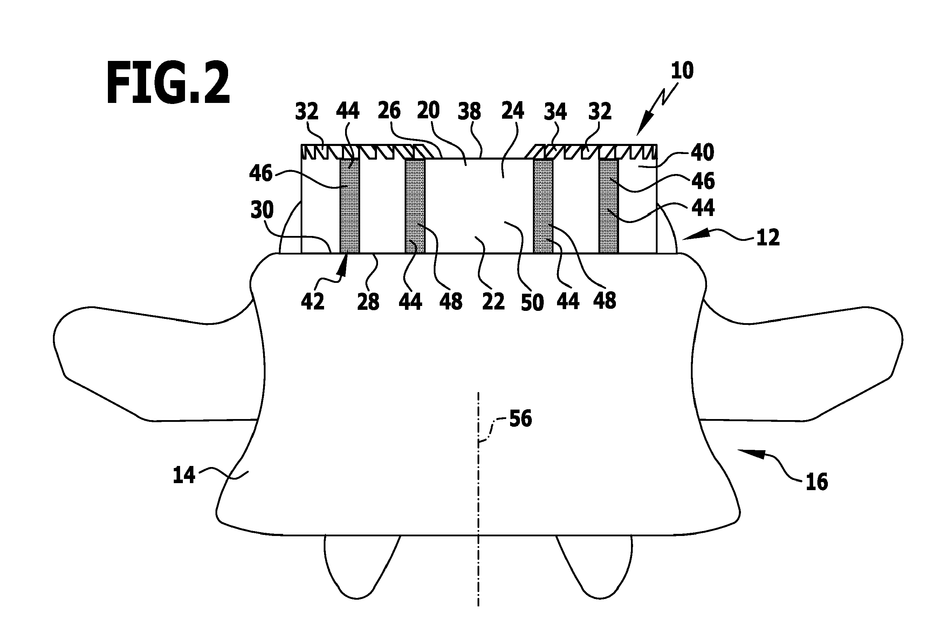

Wie schematisch in Figur 2 dargestellt, ist die Markerbeschichtung 44 unter Beaufschlagung mit Röntgenstrahlen 54 im Röntgenbild sichtbar. Da nur ein kleiner Teil der Seitenfläche 40 mit der Markerbeschichtung 44 bedeckt ist, bleibt noch ein großer Teil des Grundkörpers 24 unverdeckt, so dass ein Einwachsen von Knochen in das Implantat 10 ohne weiteres im weiteren Heilungsverlauf durch Aufnahme von Röntgenbildern verfolgt werden kann. Aus der Position der Streifen 46 und 48 in der Röntgenaufnahme kann sowohl eine Lage als auch eine Orientierung des Implantats 10 im Zwischenwirbelraum 12 ermittelt werden. In der in Figur 2 dargestellten Frontalaufnahme liegen die Streifen 48 zwischen den Streifen 46, denn die schematisch dargestellte Röntgenaufnahme stellt eine Projektion der Wirbelsäule in einer Ebene senkrecht zur Medianebene 56 dar.

## Patentansprüche

1. Implantat (10) zum Ersetzen eines Wirbelkörpers (14) oder zum Einsetzen in einen Zwischenwirbelraum (12) zwischen benachbarte Wirbelkörper (14) einer menschlichen oder tierischen Wirbelsäule (18), mit einer ersten Anlagefläche (26) zum Anlegen an eine Gelenkfläche (30) eines ersten Wirbelkörpers (14) und mit einer zweiten Anlagefläche (28) zum Anlegen an eine Gelenkfläche (30) eines zweiten Wirbelkörpers (14), wobei das Implantat (10) mindestens teilweise aus einem röntgentransparenten Material hergestellt ist und mindestens einen Röntgenmarker (42) umfasst, **dadurch gekennzeichnet, dass** der mindestens eine Röntgenmarker (42) in Form einer Metall als Markermaterial enthaltenden, mindestens teilweisen Markerbeschichtung (44) einer Seitenfläche (40) des Implantats (10), welche relativ zur ersten und/oder zweiten Anlagefläche (26, 28) um einen Neigungswinkel geneigt ist, ausgebildet ist.

2. Implantat nach Anspruch 1, **dadurch gekennzeichnet, dass** das röntgentransparente Material ein Kunststoff ist.

3. Implantat nach Anspruch 2, **dadurch gekennzeichnet, dass** der Kunststoff Polyetheretherketon (PEEK) ist oder enthält.

4. Implantat nach einem der voranstehenden Ansprüche, **dadurch gekennzeichnet, dass** der Neigungswinkel einen Wert in einem Bereich von 45° bis 135° aufweist.

5. Implantat nach Anspruch 4, **dadurch gekennzeichnet, dass** der Neigungswinkel einen Wert in einem Bereich von 70° bis 110° aufweist.

6. Implantat nach einem der voranstehenden Ansprüche, **dadurch gekennzeichnet, dass** der mindestens eine Röntgenmarker (42) in Form eines regelmäßigen oder unregelmäßigen Musters ausgebildet ist.

7. Implantat nach einem der voranstehenden Ansprüche, **dadurch gekennzeichnet, dass** der mindestens eine Röntgenmarker (42) in Form mindestens eines Buchstabens, einer Zahl und/oder eines Codes ausgebildet ist.

8. Implantat nach einem der voranstehenden Ansprüche, **dadurch gekennzeichnet, dass** mehrere Röntgenmarker (42) vorgesehen sind.

9. Implantat nach Anspruch 7, **dadurch gekennzeichnet, dass** die mehreren Röntgenmarker (42) mindestens teilweise auf unterschiedlichen Seitenflächen (40) und/oder unterschiedlichen Seiten (50, 52) vorgesehen sind.

10. Implantat nach einem der voranstehenden Ansprüche, **dadurch gekennzeichnet, dass** das Markermaterial Titan enthält.

11. Implantat nach einem der voranstehenden Ansprüche, **dadurch gekennzeichnet, dass** die Markerbeschichtung (44) aus Reintitan hergestellt ist.

12. Implantat nach einem der voranstehenden Ansprüche, **dadurch gekennzeichnet, dass** die Markerbeschichtung (44) durch Kaltgasspritzen hergestellt ist.

13. Implantat nach Anspruch 12, **dadurch gekennzeichnet, dass** eine Teilchengeschwindigkeit durch Kaltgasspritzen aufgebrachter Pulverteilchen des Markermaterials in einem Bereich von 600 m/s bis 1000 m/s liegt.

14. Implantat nach Anspruch 13, **dadurch gekennzeichnet, dass** eine Strahltemperatur beim Kaltgasspritzen in einem Bereich von 250°C bis 700°C liegt.

15. Implantat nach einem der voranstehenden Ansprüche, **dadurch gekennzeichnet, dass** die Markerbeschichtung (44) eine Dicke in einem Bereich von 0,1 mm bis 0,3 mm aufweist.

16. Implantat nach einem der voranstehenden Ansprüche, **dadurch gekennzeichnet, dass** die erste und/oder die zweite Anlagefläche (26, 28) strukturiert sind.

17. Implantat nach einem der voranstehenden Ansprüche, **dadurch gekennzeichnet, dass** die erste und/oder die zweite Anlagefläche (26, 28) Vorsprünge (34) und/oder Ausnehmungen aufweisen.

18. Implantat nach einem der voranstehenden Ansprüche, **dadurch gekennzeichnet, dass** das Implantat (10) ein erstes Anlageelement (20) und ein zweites Anlageelement (22) umfasst, dass das erste Anlageelement (20) die erste Anlagefläche (26) umfasst und dass das zweite Anlageelement (22) die zweite Anlagefläche (28) umfasst.

19. Implantat nach Anspruch 18, **dadurch gekennzeichnet, dass** das erste und das zweite Anlageelement (20, 22) unbeweglich miteinander verbunden sind.

20. Implantat nach Anspruch 18 oder 19, **gekennzeichnet durch** einen Grundkörper (24), welcher das erste und das zweite Anlageelement (20, 22) umfasst und einstückig ausgebildet ist.

21. Implantat nach Anspruch 20, **dadurch gekennzeichnet, dass** der Grundkörper (24) teilweise hohl ist und/oder mindestens eine Ausnehmung (36) aufweist.

22. Implantat nach Anspruch 18, **dadurch gekennzeichnet, dass** das erste und das zweite Anlageelement relativ zueinander beweglich gelagert sind.

23. Implantat nach Anspruch 22, **dadurch gekennzeichnet, dass** das erste und das zweite Anlageelement relativ zueinander verschwenkbar und/oder verschiebbar gelagert sind.

24. Implantat nach einem der voranstehenden Ansprüche, **dadurch gekennzeichnet, dass** die erste und/oder die zweite Anlagefläche (26, 28) mindestens teilweise mit einer osteointegrativen Beschichtung (38) versehen sind.

25. Implantat nach Anspruch 24, **dadurch gekennzeichnet, dass** die osteointegrative Beschichtung (38) porös ist.

26. Implantat nach Anspruch 24 oder 25, **dadurch gekennzeichnet, dass** die osteointegrative Beschichtung (38) eine Titan enthaltende Beschichtung ist.

27. Implantat nach Anspruch 26, **dadurch gekennzeichnet, dass** die osteointegrative Beschichtung (38) eine Rein-Titan-Beschichtung ist.

28. Implantat nach einem der Ansprüche 24 bis 27, **dadurch gekennzeichnet, dass** die osteointegrative Beschichtung (38) durch Kaltgasspritzen hergestellt ist.

29. Implantat nach einem der Ansprüche 24 bis 28, **dadurch gekennzeichnet, dass** die osteointegrative Beschichtung (38) eine Dicke in einem Bereich von 0,2 mm bis 0,5 mm aufweist.

30. Implantat nach einem der Ansprüche 24 bis 29, **dadurch gekennzeichnet, dass** die osteointegrative Beschichtung (38) dicker ist als die Markerbeschichtung (44).
